(19) Europäisches Patentamt European Patent Office Office européen des brevets

(11) **EP 2 246 712 A2**

(12) **EUROPEAN PATENT APPLICATION**

(43) Date of publication:
**03.11.2010 Bulletin 2010/44**

(51) Int Cl.:
***G01T 1/29*** *(2006.01)*

(21) Application number: **10159999.1**

(22) Date of filing: **15.04.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **16.04.2009 JP 2009099649**

(71) Applicants:
• **Kabushiki Kaisha Toshiba Minato-ku Tokyo (JP)**
• **Toshiba Medical Systems Corporation Otawara-shi, Tochigi (JP)**

(72) Inventors:
• **Takayama, Takuzo Tochigi (JP)**
• **Teshigawara, Manabu Tochigi (JP)**
• **Umehara, Takaya Tochigi (JP)**
• **Komori, Tomoyasu Tochigi (JP)**

(74) Representative: **Kramer - Barske - Schmidtchen European Patent Attorneys Landsberger Strasse 300 80687 München (DE)**

(54) **Positron emission tomography apparatus and nuclear medical image generating method**

(57) In a case that a gamma ray has entered into a plurality of scintillators adjacent to each other simultaneously, a detector detects the gamma ray having entered simultaneously. A position calculator calculates the ratio of wave heights representing the energies of the detected gamma ray. The position calculator obtains a trajectory of such a gamma ray that a ratio of distances passed by the gamma ray inside the plurality of scintillators, respectively, coincides with the ratio of the wave heights. The position calculator obtains an intersection between the boundary of the plurality of scintillators and the trajectory, as a passing position of the gamma ray. A reconstructing part executes a back projection process with the trajectory passing through the calculated passing position as a projection position.

FIG. 2

**Description**

BACKGROUND OF THE INVENTION

1. Field of the Invention

The present invention relates to a positron emission tomography apparatus that detects gamma rays radiated from radioactive isotopes inside a subject, and also relates to a nuclear medical image generating method.

2. Description of the Related Art

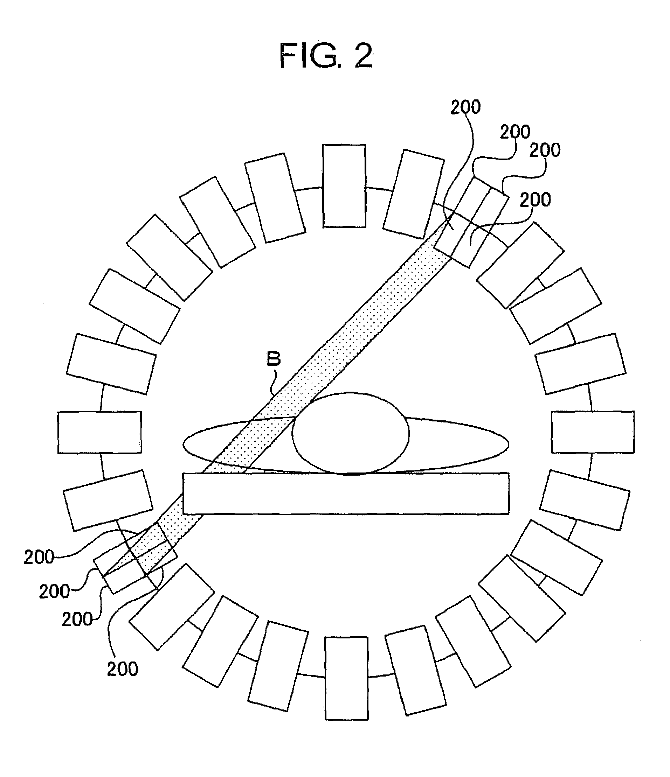

A PET apparatus detects gamma rays radiated from radioactive isotopes inside a subject, and generates an image (a nuclear medical image) based on the detection result. In an examination by the PET apparatus, positron radionuclides such as fluorodeoxyglucose (FDG) serving as radioactive isotopes are administered to the subject.
Biomolecules and the like inside the subject are labeled with the positron radionuclides as tracers. A physiological or biochemical function is obtained as data from the concentration distribution of the radioactive isotopes. This data is obtained as two-dimensional image data or three-dimensional image data.
When a positron and a free electron are bonded and annihilated, gamma rays are radiated in the opposite directions to each other. The PET apparatus detects paired gamma rays. Based on the detection result, the PET apparatus assumes that a radioactive isotope exists on a trajectory passing along the radiation directions. The PET apparatus reconstructs image data representing the concentration distribution of radioactive isotopes based on the assumption. Thus, an operator can check a lesion site, blood flow rate or fatty acid metabolism amount of the inside of the subject without using surgical means.

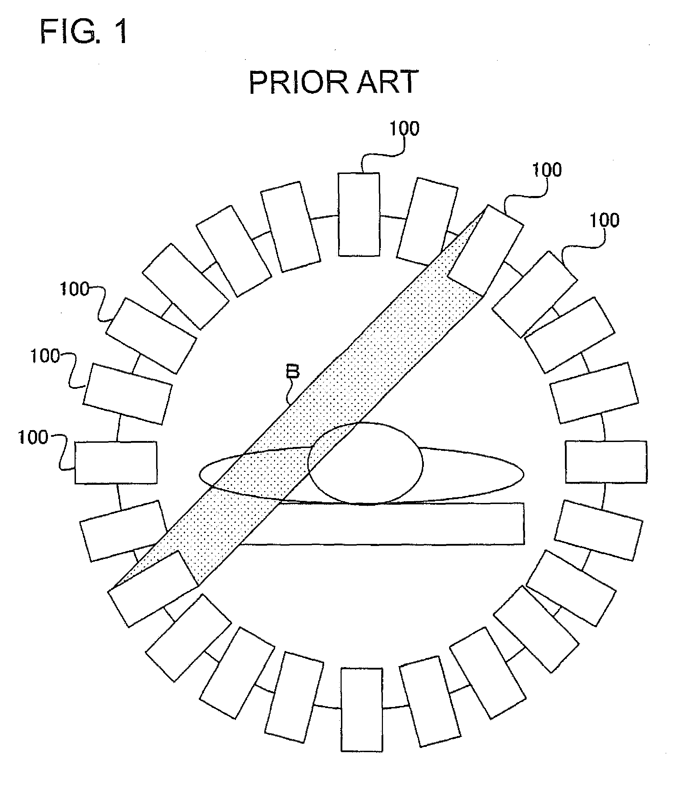

Fig. 1 shows the configuration of detectors of a PET apparatus.
The detectors are arranged in a ring shape. On each of the detectors, a plurality of scintillators 100 into which gamma rays enter are arranged on the inner circumference side of the ring. Based on paired gamma rays having entered into two of the scintillators 100 at the same timing, the PET apparatus assumes that a radioactive isotope exists on a trajectory passing through the two scintillators 100. Based on the assumption, the PET apparatus generates image data representing the concentration distribution of radioactive isotopes inside a subject P. Based on position information of the respective scintillators 100, the PET apparatus connects the positions of both the scintillators 100 into which the paired gamma rays have entered, and executes a back projection process with the connection line as a radiation trajectory of the gamma rays.
Therefore, the spatial resolution of an image depends on the size of the scintillator 100. Both the scintillators 100 into which the paired gamma rays have entered are projected in the radiation directions of the gamma rays, and a projected region shall be a projection band B. In a case that gamma rays radiated from a radioactive isotope existing at any position inside the projection band B enter into both the scintillators 100, it is impossible to specify a position inside the projection band B from which the gamma rays have been radiated.
Accordingly, the width of the projection band B determines the spatial resolution of an image.
Further, in order to increase the efficiency in detection, the scintillator 100 generally has a thickness of 20-30 mm along the depth direction. Accordingly, in a case that a gamma ray obliquely enters into the scintillator 100, the spatial resolution of an image to be obtained further decreases.
In order to increase the spatial resolution of an image, a PET apparatus in which DOI (Depth of Interaction) detectors shown in Fig. 2 are arranged in a ring shape is proposed (refer to, for example, Japanese Unexamined Patent Application Publication No. 2005-090979, and "DOI measurement apparatus" http://www.nirs.go.jp/usr/medical-imaging/ja/study/jPET_D4_2006/p87_90.pd f).
The direction of the radius of the ring is defined as the depth direction. Each of the DOI detectors is provided with a plurality of scintillators 200 stacked in the depth direction. In this PET apparatus, the projection band B is limited to the surrounding of a radiation source, and therefore, the spatial resolution of an image increases.
However, since the DOI detector is provided with the plurality of scintillators 200 arranged in the depth direction, it takes much cost.

SUMMARY OF THE INVENTION

An object of the present invention is to provide a PET apparatus capable of increasing the spatial resolution of an image while reducing the cost, and also provide a nuclear medical image generating method.
In a first aspect of the present invention, a positron emission tomography apparatus that generates an image based on a result of detection of a gamma ray radiated from each of radioactive isotopes inside a subject comprises: detectors each having a plurality of scintillators each converting the entering gamma ray into a light of a light amount corresponding to an energy of the gamma ray, the detectors being arranged in a ring shape so as to surround the subject; a position calculator configured to, when the gamma ray has entered into adjacent scintillators of the plurality of scintillators simultaneously in one detector of the detectors and the detector performs detection of the gamma ray having entered simultaneously, calculate a passing position of the gamma ray based on a result of the detection; and a reconstructing part configured to execute a back projection process with a trajectory passing through the calculated passing position as a projection direction to reconstruct the image of concentration distribution of the radioactive isotopes inside the subject.

According to the first aspect, in a case that the gamma ray has entered into the adjacent scintillators simultaneously, the passing position of the gamma ray is calculated based on the result of the detection by the detector. Thus, it is possible to make the spatial resolution of an image higher than the conventional PET apparatus while reducing the cost as compared with the DOI detector.

A second aspect of the present invention is a nuclear medical image generating method of, by each of detectors arranged in a ring shape so as to surround a subject, performing detection of a gamma ray radiated from each of radioactive isotopes inside the subject, and generating an image based on a result of the detection by the detectors.

The detectors each have a plurality of scintillators each converting the entering gamma ray into a light of a light amount corresponding to an energy of the gamma ray. When the gamma ray has entered into adjacent scintillators of the plurality of scintillators simultaneously in one detector of the detectors and the detector has performed detection of the gamma ray having entered simultaneously, a passing position of the gamma ray is calculated based on a result of the detection. By execution of a back projection process with a trajectory passing through the calculated passing position as a projection direction, the image of the concentration distribution of the radioactive isotopes inside the subject is reconstructed.

## BRIEF DESCRIPTION OF THE DRAWINGS

Fig. 1 shows a detector of a conventional PET apparatus.
Fig. 2 shows a DOI detector.
Fig. 3 shows the configuration of a PET apparatus.
Fig. 4 shows the configuration of a detector included in the PET apparatus.
Fig. 5 shows a detection pattern in a case that a gamma ray enters into one scintillator.
Fig. 6 shows a detection pattern in a case that a gamma ray enters so as to pass through two adjacent scintillators.
Fig. 7 shows a second detection pattern in a case that a gamma ray enters so as to pass through two adjacent scintillators.
Fig. 8 shows a detection pattern in a case that a gamma ray enters so as to pass through three adjacent scintillators.
Fig. 9 is a graph showing the number of gamma rays with respect to the depth direction of scintillators.
Fig. 10 is a flow chart showing an example of the operation of the PET apparatus.
Fig. 11 is a flow chart showing the example of the operation of the PET apparatus.
Fig. 12 shows a list of single information.

## DETAILED DESCRIPTION OF THE EMBODIMENTS

A PET apparatus and a nuclear medical image generating method according to an embodiment of the present invention will be described with reference to the drawings.

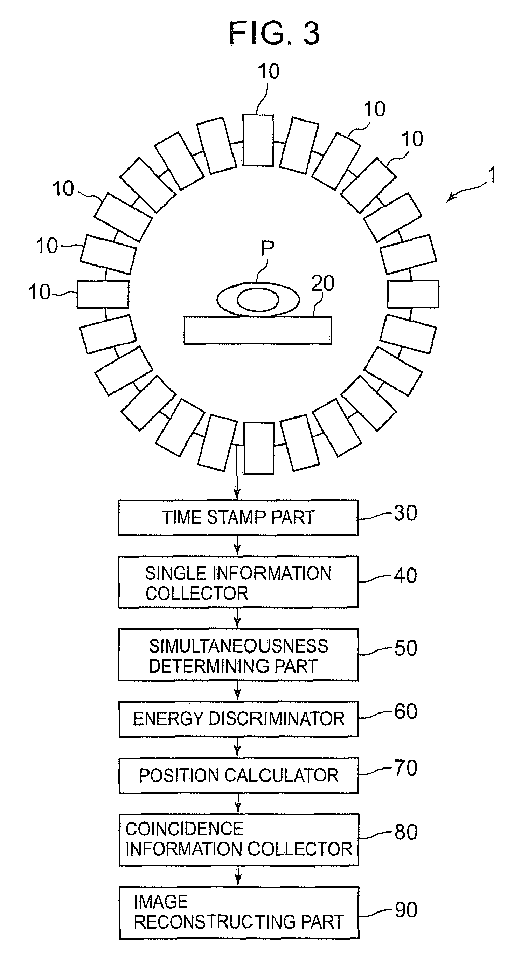

A PET apparatus 1 shown in Fig. 3 detects gamma rays radiated from radioactive isotopes inside a subject P. The PET apparatus 1 generates an image of the inside of the subject P based on the detection result.

As the radioactive isotopes, positron radionuclides such as fluorodeoxyglucose are used.

When a positron emitted from a positron radionuclide and a near free electron are bonded and annihilated, gamma rays of 511 keV are radiated in the opposite directions to each other (the opposite directions at 180 degrees). The PET apparatus 1 detects the gamma rays. The PET apparatus 1 discriminates gamma rays having entered at the same timing to determine the entering directions of the gamma rays.

Assuming that a radioactive nuclide exists on a trajectory of the entering directions, the PET apparatus 1 reconstructs an image by a back projection process. The entering direction of a gamma ray subjected to Compton scattering before entering may be different from the radiation direction. The PET apparatus 1 increases the accuracy of determination of the entering direction by discriminating a gamma ray having an energy of the Compton edge or more, other than considering the entrance timing.

The image generated by the PET apparatus 1 is data in which the degree of overlap of trajectories passing along the entering directions of gamma rays is expressed by pixel values. This image represents the concentration distribution of radioactive isotopes inside the subject P.

The PET apparatus 1 is provided with a plurality of detectors 10, a bed 20, a time stamp part 30, a single information collector 40, a simultaneousness determining part 50, an energy discriminator 60, a position calculator 70, a coincidence information collector 80, and an image reconstructing part 90.

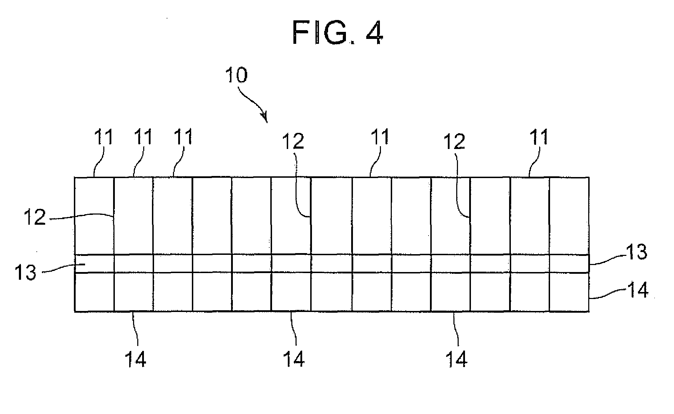

The plurality of detectors 10 are arranged in a ring shape. As shown in Fig. 4, each of the detectors 10 is provided with a plurality of scintillators 11 arranged on the inner circumference side of the ring.

The detector 10 is a pixel-type detector. The scintillators 11 convert entering gamma rays into lights of light amounts corresponding to the energies of the gamma rays.

The area of each of the scintillators 11 is, for example, 4-mm-square or 1.6-mm-square. The depthwise thickness of the scintillator 11 is preferably 20 mm or less, more preferably 10 mm or less. As the scintillator 11, such crystal that is high in detection efficiency for a gamma ray having an energy of 511 keV and that is not deliquescent is used. For example, LSO ($Lu_2SiO_5$) BGO ($Bi_4Ge_3O_{12}$) or the like is used as the scintillator. Reflectors 12 are placed between the respective scintillators 11. By the reflectors 12, the scintillators 11 are separated, respectively.

On the back of each of the scintillators 11, a light guide 13 is placed for every scintillator 11. Moreover, on the back of each of the scintillators 11, a photomultiplier tube (PMT) 14 is placed across the light guide 13 for every scintillator 11. That is to say, the detector 10 is provided with the plurality of scintillators 11, the plurality of light guides 13, and the plurality of photomultiplier tubes 14. On each of the scintillators 11, the light guide 13 and the photomultiplier tube 14 are placed. The photomultiplier tube 14 converts a light into an electric signal corresponding to the amount of the light. The photomultiplier tube 14 is a position sensitive photomultiplier tube. The photomultiplier tubes 14 convert lights outputted by the scintillators 11 into electric signals in accordance with the amounts of the lights, respectively. Instead of the photomultiplier tube 14, a position sensitive silicon detector may be used.

The detector 10 converts gamma rays having entered into the scintillators 11 into lights having the peak around the wavelength of 420 nm. The detector 10 converts the lights into electric signals corresponding to the amounts of the lights and outputs the electric signals by the photomultiplier tubes 14. Each of the electric signals contains position information representing the position of the scintillator 11 into which the gamma ray has entered, and energy information representing the energy of the gamma ray having been converted into the light by the scintillator 11. The energy information represents the wave height of the electric signal.

On the bed 20, the subject P is placed. The subject P is inserted into the ring of the detectors 10. To the subject P, radioactive isotopes are administered in advance. The detectors 10 surround the subject P like a ring and detect gamma rays radiated from inside the subject P.

The time stamp part 30 has a timer. The time stamp part 30 stamps detection timing information representing a time when the electric signal outputted from the detector 10 has been detected.

The single information collector 40 includes a memory. The memory stores all single information generated during a period between the start of detection of gamma rays and the end of the detection. The single information is data in which the electric signals outputted from the detectors 10 and the time stamp part 30 are digitalized via an analog-digital converter. That is to say, the single information contains the energy information of the gamma ray having entered, the position information representing the position of the scintillator 11 into which the gamma ray has entered, and the detection timing information. The energy information of the gamma ray is such a value that a peak value e representing the energy the gamma ray is converted into a digital peak value e such as 10 bits.

The simultaneousness determining part 50 selects single information whose detection timings are simultaneous, and outputs a group of the selected single information to the energy discriminator 60.

"Being simultaneous" means a case that the difference in time of the timings represented in the detection timing information is, for example, within about 10 ns. The simultaneousness determining part 50 previously holds a timing window showing a predetermined time difference. The simultaneousness determining part 50 outputs a group of single information fallen within the timing window to the energy discriminator 60.

The group of the single information discriminated by the simultaneousness determining part 50 contains single information generated as a result that paired gamma rays enter into the two scintillators 11 opposite to each other, respectively. Moreover, the group of the single information also contains a plurality of single information generated as a result that a gamma ray crosses the plurality of adjacent scintillators 11 and that some photoelectric effects, Compton scattering, and electron pair production occur on a trajectory L of the gamma ray.

The energy discriminator 60 outputs, to the position calculator 70, a group of single information excluding the group containing the single information resulting from detection of a gamma ray subjected to Compton scattering caused before entering into the scintillator 11.

The energy discriminator 60 holds energy range information representing the range of energy in advance. This energy range is a range that is equal to or more than the Compton edge and that includes 511 keV.

In a case that a gamma ray enters into only one of the scintillators 11 in one of the detectors 10, the energy discriminator 60 determines whether the energy value represented by single information resulting from detection by this scintillator 11 is included in a range represented by energy range information.

In a case that a gamma ray enters into the adjacent scintillators 11 simultaneously in one of the detectors 10, the energy discriminator 60 calculates the sum of energies represented by the respective single information resulting from detection by the respective scintillators 11.

The energy discriminator 60 determines whether the sum of the energies is included in a range represented by the energy range information. If the sum of the energies of the gamma ray due to all photoelectric effects, Compton scattering, and electron pair production having occurred within the adjacent scintillators 11 is equal to or more than the Compton edge, the detection result by each of the scintillators 11 is not based on the occurrence of Compton scattering before entrance into the detector 10. A case that a gamma ray enters into the adjacent scintillators 11 in one of the detectors 10 is equivalent to a case that the trajectory L of the gamma ray crosses the scintillators 11.

To be specific, for the adjacent scintillators 11, the energy discriminator 60 sums the peak values e represented by the energy information of the single information, respectively. The energy discriminator 60 determines whether all of the sums are included in the range represented by the energy range information. In a case that all of the determination results on a certain group are included in the range represented by the energy range information, the energy discriminator 60 outputs the group of the single information to the position calculator 70.

The position calculator 70 calculates positions through which gamma rays radiated in the opposite directions to each other (the opposite directions at 180 degrees) pass. In a case that a gamma ray enters into the adjacent scintillators 11 simultaneously in one of the detectors 10, the position calculator 70 calculates the passing positions of the gamma ray by using the position information and energy information of these scintillators 11. A case that a gamma ray enters into the plurality of adjacent scintillators 11 simultaneously in one of the detectors 10 is a case that a plurality of single information having a relation in which the positions represented by position information are adjacent to each other exist in the same group.

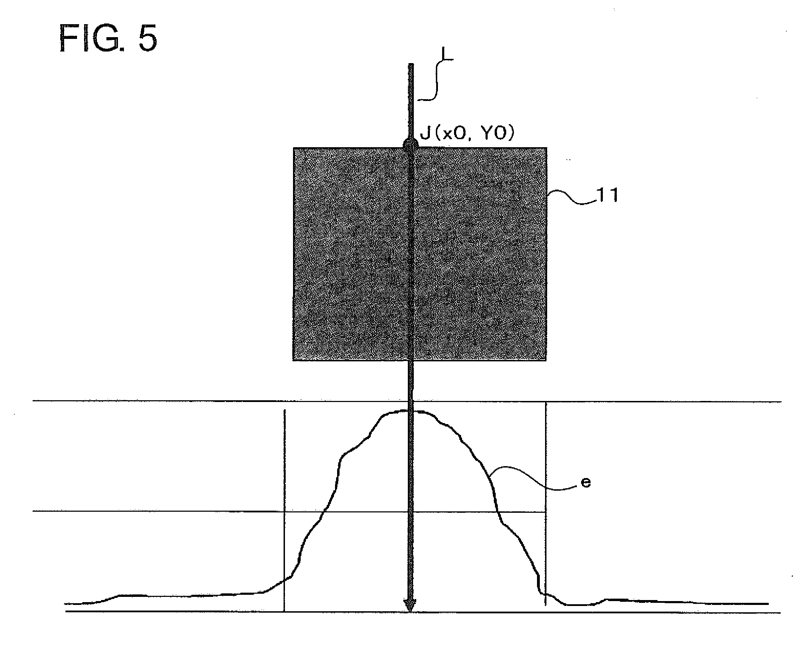

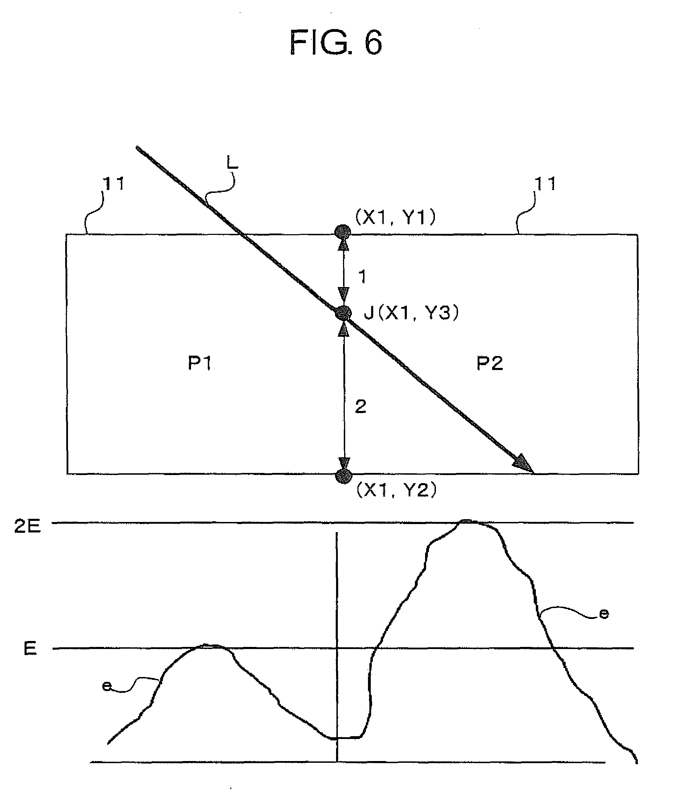

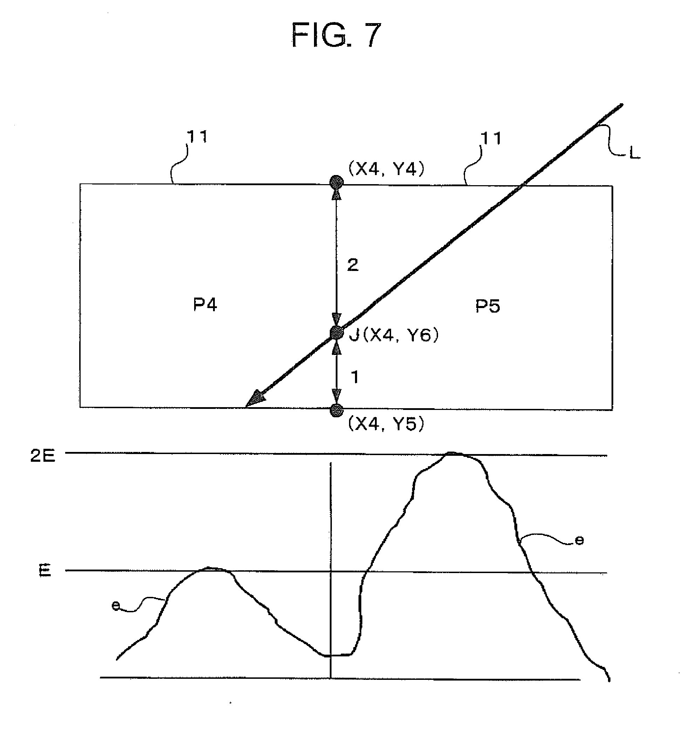

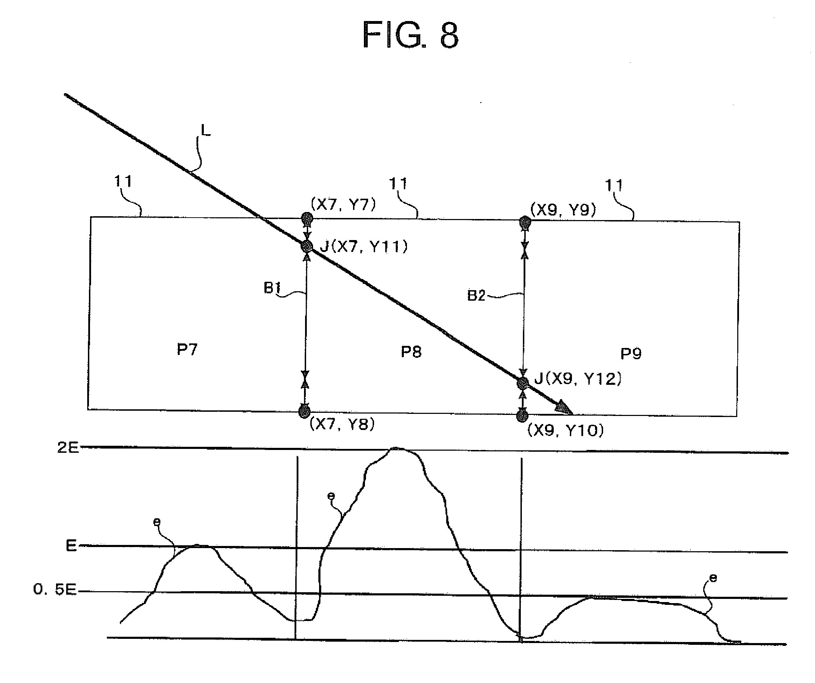

Figs. 5-8 show the concept of calculation by the position calculator 70. Fig. 5 shows a case that a gamma ray enters into only one of the scintillators 11 in one of the detectors 10. Fig. 6 shows a case that a gamma ray enters so as to pass through two of the scintillators 11 adjacent to each other in one of the detectors 10. Fig 7 shows a case that the entering direction of a gamma ray is opposite to the entering direction in Fig. 6. In Fig. 7, the energies of the gamma ray entering into the respective scintillators 11 shall be identical to each other. Fig. 8 shows a case that a gamma ray enters so as to pass through three of the scintillators 11 adjacent to each other in one of the detectors 10.

A case that a gamma ray enters into only one of the scintillators 11 in one of the detectors 10 as shown in Fig. 5 will be described. That is to say, a case that there are no such single information that positions represented by position information are in an adjacent relation in the same group will be described.

The position calculator 70 generates new single information by combining passing position information that is held in advance in accordance with the position information contained in the single information, with the energy information and detection timing information that are contained in the single information. The passing position information held in advance is, for example, the coordinates of the center of the scintillator 11 into which the gamma ray has entered.

A case that a gamma ray enters into the two adjacent scintillators 11 simultaneously in one of the detectors 10 as shown in Fig. 6 will be described. That is to say, a case that there are two such single information that positions represented by position information are in an adjacent relation in the same group will be described.

The position calculator 70 calculates the ratio of the peak values e of the gamma ray having entered into the two adjacent scintillators 11, respectively, based on energy information resulting from detection by each of the two adjacent scintillators 11. The position calculator 70, when the gamma ray passes across the two adjacent scintillators 11, obtains such a trajectory L that the ratio of passing distances of the gamma ray in the respective scintillators 11 coincides with the ratio of the peak values e. The position calculator 70 obtains, as a passing position, an intersection (a division point) J between the boundary of the two adjacent scintillators 11 and the trajectory L. In this embodiment, in a case that forward scattering of Compton scattering occurs with the same probability at each point along the entering direction of the gamma ray, it is inferred that the peak value of the energy information is proportion to the passing distance. It is inferred that the gamma ray passes through this passing position when entering into the scintillator 11 at any angle. The position calculator 70 obtains the intersection (the division point) J based on this inference.

The position calculator 70 previously holds coordinate information of the boundary upper end and boundary lower end of the two adjacent scintillators 11. The position calculator 70 calculates the ratio of the peak values e represented by both the energy information.

The position calculator 70 obtains the coordinate information of the division point J by dividing a line segment held between the boundary upper end and the boundary lower end at the ratio of the peak values e. The position calculator 70 defines the coordinate information of the division line J as passing position information, and defines the sum of both the energy information as new energy information. The position calculator 70 generates the new single information by combining the passing position information, the new energy information, and one of the detection timing information contained in both the single information.

Even if a gamma ray enters into the two adjacent scintillators 11 from different directions as shown in Figs. 6 and 7, the outputted energy information may be the same. Before calculating the coordinate information of the division line J, the position calculator 70 schematically discriminates a direction in which one of paired gamma rays has entered into the two adjacent scintillators 11 by using position information of the scintillator 11 into which the other gamma ray has entered.

The position calculator 70 changes an application pattern of the ratio in the division, depending on which side of regions separated by a line connecting the two adjacent scintillators 11 and the scintillators 11 placed opposite to the two adjacent scintillators 11, a position represented by the position information contained in the single information generated as a result that the other gamma ray of the paired gamma rays has been detected is included in. The application pattern of the ratio is division at a ratio of a:b or division at a ratio of b:a from the boundary upper end. For example, the position calculator 70 can obtain the application pattern of the ratio by comparing the magnitudes of the position represented by the position information of the scintillators 11 placed opposite to the two adjacent scintillators 11 and of the position represented by the position information contained in the single information generated as a result that the other gamma ray of the paired gamma rays has been detected.

After the schematic discrimination of the entering direction, the position calculator 70 obtains a ratio shown below. By using the ratio, the position calculator 70 divides the boundary of the two adjacent scintillators 11 along the depth direction to obtain the coordinate information of the division point J.

The ratio is expressed as follows:

(the peak value e of energy of the gamma ray having entered into the scintillator 11 placed on the front side in the entering direction) : (the peak value e of energy of the gamma ray having entered into the scintillator 11 placed on the rear side).

A case that a gamma ray has entered into the three adjacent scintillators 11 in one of the detectors 10 simultaneously as shown in Fig. 8 will be described.

The position calculator 70 calculates the ratio of peak values e of energies of the gamma ray having entered into the three adjacent scintillators 11, respectively, based on energy information resulting from detection by each of the three adjacent scintillators 11. The position calculator 70, when the gamma ray passes across the three adjacent scintillators 11, obtains a trajectory L that the ratio of passing distances of the gamma ray in the respective scintillators 11 coincides with the ratio of the peak values e. The position calculator 70 obtains, as passing positions, intersections (division points) J between the boundaries of the three adjacent scintillators 11 and the trajectory L.

The position calculator 70 holds, in advance, coordinate information of the boundary upper end and boundary lower end of the two adjacent scintillators 11. The position calculator 70 calculates the ratio of the peak values e represented by three energy information. The position calculator 70 divides the respective line segments held between the boundary upper end and the boundary lower end at the ratio of the peak values e. Although two division points J are obtained on each of the boundaries, the passing position of the gamma ray is one point for each of the boundaries. The position calculator 70 schematically discriminates the entering direction of the gamma ray.

The position calculator 70 selects the division point J on the upper end side of the boundary located on the front side in the entering direction and the division point J on the lower end side of the boundary located on the rear side in the entering direction, respectively. The position calculator 70 defines the two selected division points as the passing positions of the gamma ray. Alternatively, the position calculator 70 may define one of the selected division points as the passing position of the gamma ray.

After calculation of the passing position of the gamma ray in the same group, the position calculator 70 generates coincidence information by combining passing position information, energy information, and detection timing information. The position calculator 70 outputs the coincidence information to the coincidence information collector 80. The coincidence information is data that contains passing position information representing two or more passing positions on the trajectory L of paired gamma rays, respectively, energy information representing energies of both the paired gamma rays, and detection timing information of the paired gamma rays.

The coincidence information collector 80 includes a memory.

The coincidence information collector 80 stores all coincidence information generated during a period between the start and end of detection of gamma rays.

The image reconstructing part 90 counts all coincidence information containing the same passing position information. The image reconstructing part 90 generates projection data in which the counted number and the passing position information are associated.

The image reconstructing part 90 generates an image by executing a back projection process on the projection data. As the back projection process, the filtered back projection method, the OS-EM (Ordered Subset Expectation Maximization) reconstruction method, and the like may be applied. By the back projection method, the image reconstructing part 90 generates an image in which the concentration distribution of radioactive isotopes inside the subject P is shown.

In the above example, the position calculator 70 infers that the peak value e of energy is proportion to the passing distance to obtain the passing position (the division point J) of the gamma ray.

The position calculator 70 may obtain the passing position of the gamma ray by another method without using this inference.

For example, the position calculator 70 may obtain the passing position of the gamma ray, based on the energy of the gamma ray having entered into the adjacent scintillators 11 and based on stopping power $\alpha$ of each of the scintillators 11.

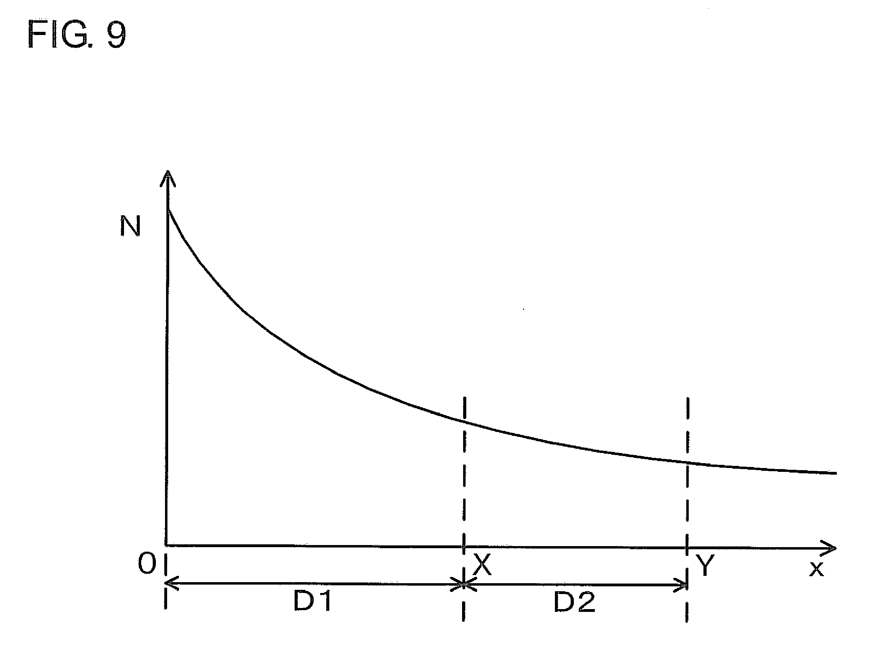

With reference to Fig. 9, a method for obtaining the passing position of the gamma ray by using the stopping power $\alpha$ will be described. As one example, a case that the gamma ray has entered into the two adjacent scintillators 11 simultaneously in one of the detectors 10 as shown in Fig. 6 will be described.

Fig. 9 is a graph showing the number of gamma rays with respect to the depth direction (the x direction) of the scintillators 11.

The horizontal axis (the x axis) takes the depth direction of the scintillators 11. The vertical axis (N) takes the number of gamma rays.

In Fig. 9, a position X in the depth direction is equivalent to a position on the boundary of the two adjacent scintillators 11, which is a position of a depth D1 from the upper end of the boundary. The sum of the depth D1 and a depth D2 is equivalent to the thickness in the depth direction of each of the scintillators 11.

The gamma ray having entered into the scintillator 11 gradually decreases in the depth direction of the scintillator 11, in accordance with the stopping power $\alpha$ that depends on the material of the scintillator 11.

The loss of the energy of the gamma ray in the two scintillators 11 is expressed by the following equation (1).

Equation (1)

(the energy of the gamma ray having entered into the first scintillator 11) : (the energy of the gamma ray having entered into the second scintillator 11)

$$= \left( \int_0^X e^{-\frac{1}{\alpha}x} \, dx \right) : \left( \int_X^Y e^{-\frac{1}{\alpha}x} \, dx \right)$$

$$= \left[ -\alpha \cdot e^{-\frac{1}{\alpha}x} \right]_0^X : \left[ -\alpha \cdot e^{-\frac{1}{\alpha}x} \right]_X^Y$$

Reference symbol $\alpha$ denotes stopping power determined by the material of the scintillator 11. The stopping power $\alpha$ is a known value.

The first scintillator 11 is equivalent to the scintillator 11 placed on the left side in Fig. 6. The second scintillator 11 is equivalent to the scintillator 11 placed on the right side in Fig. 6.

The above equation (1) shows the ratio of the loss of the energy of the gamma ray in the first scintillator 11 and the loss of the energy of the gamma ray in the second scintillator 11.

That is to say, the equation (1) shows a state as shown in Fig. 6 that the gamma ray enters into the scintillator 11 placed on the left side (the first scintillator 11), exits from the first scintillator 11, and then enters into the scintillator 11 placed on the right side (the second scintillator 11).

The position calculator 70 obtains the position X in the depth direction on the boundary, based on the equation (1) expressed using the stopping power $\alpha$ and based on the energy of the gamma ray having entered into each of the scintillators 11.

As mentioned above, the single information contains the position information representing the position of the scintillator 11 into which the gamma ray has entered, and the energy information representing the energy of the gamma ray.

The position calculator 70 obtains the position of the depth D1 on the boundary by substituting the position information and energy information contained in the single information into the equation (1).

To be specific, the position calculator 70 obtains the position X of the depth D1 on the boundary by substituting, into the equation (1), the energy of the gamma ray having entered into the first scintillator 11, the energy of the gamma ray having entered into the second scintillator 11, and the stopping power $\alpha$ of the scintillator 11.

The position calculator 70 defines the position X of the depth D1 on the boundary as the passing position of the gamma ray.

Thus, in accordance with the theoretically expressed equation (1), the passing position of the gamma ray may be obtained. By using the stopping power $\alpha$ of the scintillator 11, it is possible to more accurately obtain the passing position of the gamma ray.

Further, even when a gamma ray enters into the three adjacent scintillators 11 simultaneously in one of the detectors 10, it is possible to obtain the passing position of the gamma ray by using the stopping power $\alpha$. That is to say, the position calculator 70 obtains the passing position of the gamma ray based on the energy of the gamma ray having entered into each of the three adjacent scintillators 11, and on the stopping power $\alpha$ of the scintillator 11.

In this embodiment, the passing position of the gamma ray may be obtained in accordance with the inference that the peak value e is proportion to the passing distance, or the passing position of the gamma ray may be obtained in accordance with the equation (1) expressed using the stopping power $\alpha$.

It is also possible to configure so that the operator can select a method of obtaining the passing position of the gamma ray with a manipulation part not shown in the drawing.

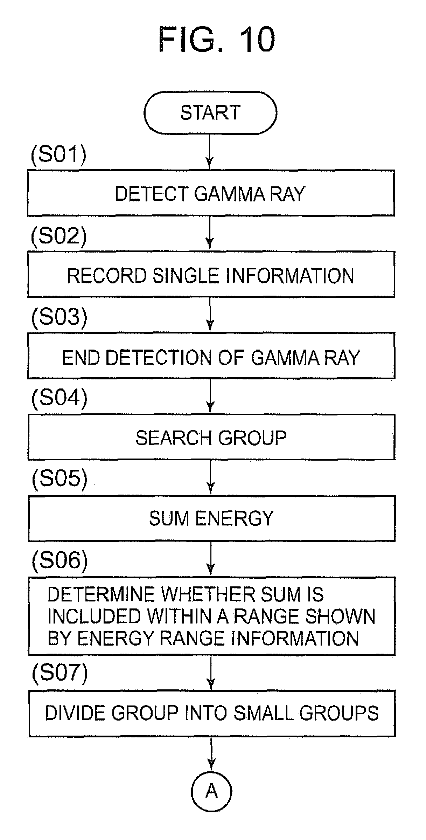

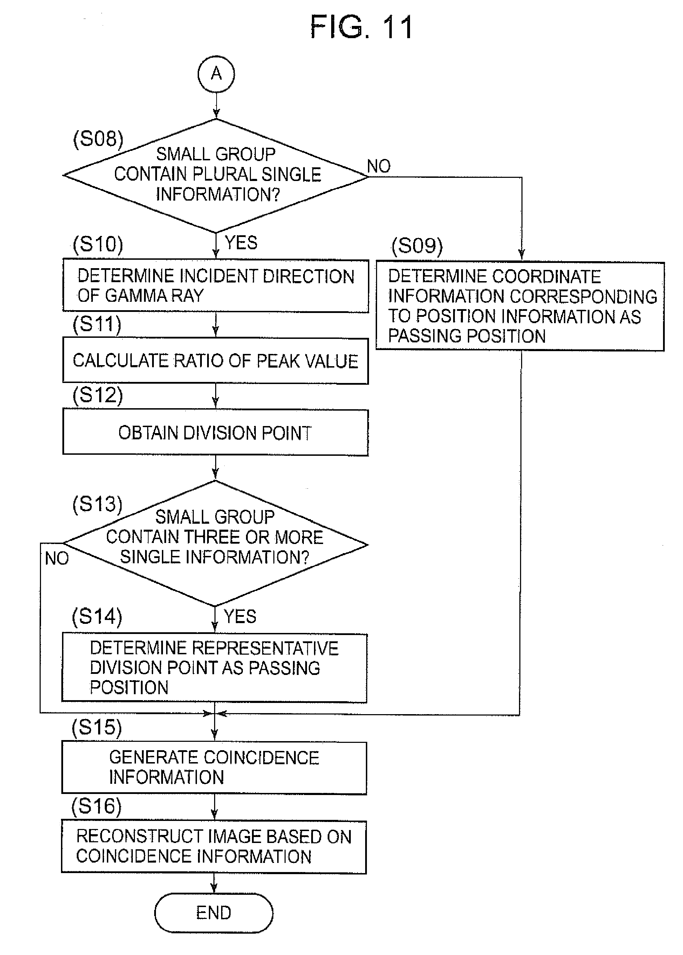

With reference to Figs. 10 and 11, one example (a method for generating a nuclear medical image) of the operation of the PET apparatus 1 will be described.

Fig. 10 shows a process from step S01 to step S07.
Fig. 11 shows a process from step S08 to step S16.

Firstly, paired gamma rays are radiated in the opposite directions to each other from inside the subject P, respectively, and the detectors 10 detect the gamma rays (S01).

Single information on which detection timing has been stamped by the time stamping part 30 is recorded into the single information collector 40 (S02).

After measuring a predetermined time period, the PET apparatus ends detection of gamma rays (S03). Alternatively, the PET apparatus may end detection of gamma rays after counting a predetermined count number.

When the detection of gamma rays ends, the simultaneousness determining part 50 searches the single information collector 40 for a group of single information included within the time window (S04).

The energy discriminator 60 extracts, from the group, a plurality of single information in which position information represent an adjacent relation. The energy discriminator 60 sums energies represented by the respective single information (S05).

The energy discriminator 60 determines whether the sum of the energies is included within a range represented by the energy range information (S06).

The position calculator 70 divides the group into small groups based on the adjacent relation represented by the position information (S07). The position calculator 70 divides the group into a small group in which the position information represents the adjacent relation and a small group in which the position represented by the position information is isolated. The position calculator 70 calculates the passing position for each of the small groups.

In a case that only one single information belongs to the small group (SO8, No), the position calculator 70 determines the coordinate information previously held in accordance with the position information contained in this single information, as the passing position of the gamma ray (S09).

In a case that a plurality of single information belong to the small group (SO8, Yes), the position calculator 70 schematically determines the entering direction of the gamma ray (S10). For example, the position calculator 70 compares position information of the scintillator 11 opposite to the scintillator 11 located in a position represented by the position information contained in any of the single information, with position information contained in the single information of the other small group. The position calculator 70 determines the entering direction of the gamma ray in accordance with the relation in magnitude of the positions represented by the position information.

The position calculator 70 calculates the ratio of the peak values e represented by the plurality of single information belonging to the small group (S11). The ratio of the peak values e is as follows:

(the peak value e of the gamma ray having entered into the scintillator 11 on the front side in the entering direction) : (the peak value e of the gamma ray having entered into the scintillator 11 on the rear side)

The position calculator 70 divides a line segment between the boundary upper end and boundary lower end held in advance at the ratio calculated at step S11 to calculate the coordinate information of the division point J. The position calculator 70 determines the division point J as the passing position (S12).

In a case that three or more single information are included in the small group (S13, Yes), the position calculator 70 selects division points one by one from the boundary on the front side toward the boundary on the rear side of the entering direction. The position calculator 70 defines any of the selected division points as a representative division point. The position calculator 70 determines the representative division point J as the passing position (S14).

After calculation of the passing position (S09, S12, S14), the position calculator 70 generates coincidence information containing the passing position information (S15). The position calculator 70 records the coincidence information into the coincidence information collector 80.

The image reconstructing part 90 executes a back projection process by using the coincidence information recorded in the coincidence information collector 80 to reconstruct an image (S16).

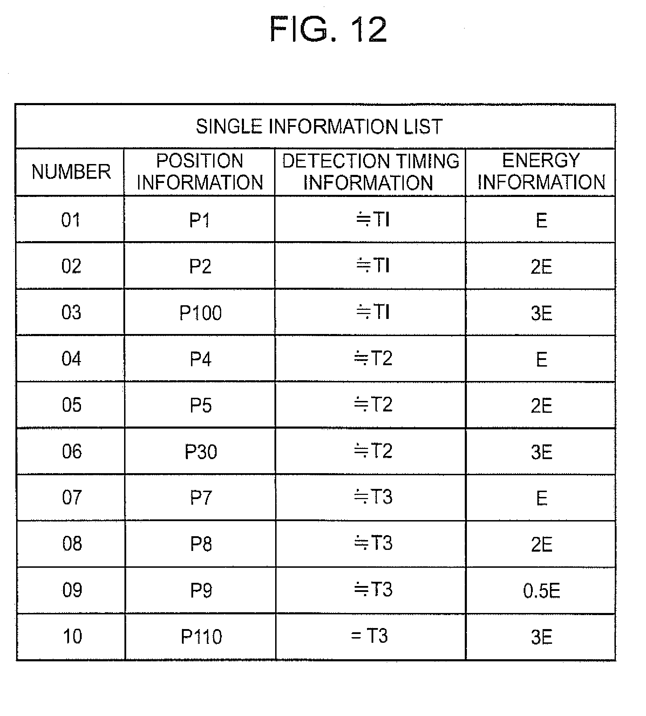

With reference to Figs. 6-8 and 12, a specific example of the process by the PET apparatus 1 will be described. Fig. 12 shows a list of single information.

Number 01, number 02, and number 03 shown in Fig. 12 represent the result of detection of paired gamma rays radiated in the opposite directions to each other (the opposite directions at 180 degrees). The respective single information of number 01 and number 02 represent the result obtained by entrance of the gamma ray into the two adjacent scintillators 11 as shown in Fig. 6. The respective single information of number 01, number 02, and number 03 contain detection timing information T1 that the detection timings of the gamma ray become almost simultaneous in the respective single information. The single information of number 01 contains position information P1. The single information of number 02 contains position information P2. The position represented by the position information P1 and the position represented by the position information P2 are in an adjacent relation.

Energy information 3E as the sum of energy information E (peak value e) of number 01 and energy information 2E (peak value e) of number 02 shall be included within a range represented by the energy range information. Moreover, energy information 3E (peak value e) of number 03 shall be included within the range represented by the energy range information.

In this case, the respective single information of number 01, number 02, and number 03 are outputted to the position calculator 70 as the same group.

The scintillator 11 placed at a position represented by position information P100 of number 03 shall be placed closer to the scintillator 11 placed at the position represented by the position information P1 than to the scintillator 11 placed at the position represented by the position information P2.

The position information P1 of number 01 and the position information P2 of number 02 represent the adjacent relation. Therefore, the position calculator 70 includes the single information of number 01 and the single information of number 02 into the same small group.

The position represented by the position information P100 of number 03 is not adjacent to either the position represented by the position information P1 or the position represented by the position information P2. Therefore, the position calculator 70 includes the single information of number 03 into another small group.

The position calculator 70 calculates the passing position of the gamma ray for each of the small groups.

The position information P100 represents that the gamma ray has entered from the side of the scintillator 11 placed at the position represented by the position information P1. Therefore, the position calculator 70 obtains a division point J (X1,Y3) that divides a line segment between coordinates (X1,Y1) of the boundary upper end and coordinates (X1,Y2) of the boundary lower end of the scintillator 11 in the depth direction at a ratio of (1:2). That is to say, the position calculator 70 calculates $Y3 = (2\times Y1+Y2)/3$. The coordinates (X1,Y1) of the boundary upper end and the coordinates (X1,Y2) of the boundary lower end of the scintillator 11 are defined by the position information P1 and the position information P2. The position calculator 70 determines the division point J (X1, Y3) as the passing position of one gamma ray of paired gamma rays represented by the respective single information of number 01, number 02, and number 03.

Number 04, number 05, and number 06 shown in Fig. 12 represent the result of detection of paired gamma rays radiated in the opposite directions to each other (the opposite directions at 180 degrees). Single information of each of number 04 and number 05 represents the result obtained by entrance of the gamma ray into the two adjacent scintillators 11 as shown in Fig. 7.

The respective single information of number 04, number 05, and number 06 contain such detection timing information T2 that the timings of detection of the gamma ray are almost simultaneous in the respective single information. The single information of number 04 contains position information P4. The single information of number 05 contains position information P5. A position represented by the position information P4 and a position represented by the position information P5 are in an adjacent relation.

Energy information 3E as the sum of energy information E (peak value e) of number 04 and energy information 2E (peak value e) of number 05 shall be included within a range represented by the energy range information. Moreover, energy information 3E (peak value e) of number 06 shall be included within the range represented by the energy range information.

In this case, the respective single information of number 04, number 05, and number 06 are outputted to the position calculator 70 as the same group.

The scintillator 11 placed at a position represented by position information P30 of number 06 shall be placed closer to the scintillator 11 placed at the position represented by the position information P5 than to the scintillator 11 placed at the position represented by the position information P4.

The position information P4 of number 04 and the position information P5 of number 05 represent an adjacent relation. Therefore, the position calculator 70 includes the single information of number 04 and the single information of number 05 into the same small group.

The position represented by the position information P30 of number 06 is not adjacent to either the position represented by the position information P4 or the position represented by the position information P5. Therefore, the position calculator 70 includes the single information of number 06 into another small group.

The position calculator 70 calculates the passing position of the gamma ray for each of the small groups.

The position information P30 represents that the gamma ray has entered from the side of the scintillator 11 placed at the position represented by the position information P5. Therefore, the position calculator 70 obtains a division point J (X4,Y6) that divides a line segment between coordinates (X4,Y4) of the boundary upper end and coordinates (X4,Y5) of the boundary lower end of the scintillator 11 in the depth direction at a ratio of (2:1). That is to say, the position calculator 70 calculates $Y6 = (Y4+2{\times}Y5)/3$. The coordinates (X4,Y4) of the boundary upper end and coordinates (X4,Y5) of the boundary lower end of the scintillator 11 are defined by the position information P4 and the position information P5. The position calculator 70 determines the division point J (X4,Y6) as the passing position of one gamma ray of paired gamma rays represented by the respective single information of number 04, number 05, and number 06.

Number 07, number 08, number 09, and number 10 shown in Fig. 12 represent the result of detection of paired gamma rays radiated in the opposite directions to each other (the opposite directions at 180 degrees). Single information of each of number 07, number 08, and number 09 represent the result obtained by entrance of the gamma ray into the three adjacent scintillators 11 as shown in Fig. 9. The respective single information of number 07, number 08, number 09, and number 10 contain such detection timing information T3 that the timings of detection of the gamma ray are almost simultaneous in the respective single information. The single information of number 07 contains position information P7. The single information of number 08 contains position information P8. The single information of number 09 contains position information P9.

Energy information (3.5E) as the sum of energy information E (peak value e) of number 07, energy information 2E (peak value e) of number 08, and energy information (0.5E) of number 09 shall be included within a range represented by the energy range information.

Moreover, energy information 3E (peak value e) of number 10 shall be included within the range represented by the energy range information.

In this case, the respective single information of number 07, number 08, number 09, and number 10 are outputted to the position calculator 70 as the same group.

The scintillator 11 placed at a position represented by position information P110 of number 10 shall be placed closer to the scintillator 11 placed at a position represented by position information P7 than to the scintillator 11 placed at a position represented by position information P9.

The position information P7 of number 07, the position information P8 of number 08, and the position information P9 of number 09 represent an adjacent relation. Therefore, the position calculator 70 includes the respective single information of number 07, number 08, and number 09 into one small group.

The position represented by the position information P110 of number 10 is not adjacent to either the position represented by the position information P7 or the position represented by the position information P9. Therefore, the position calculator 70 includes the single information of number 10 into another small group.

The position calculator 70 calculates the passing position of the gamma ray for each of the small groups.

The position information P110 represent that the gamma ray has entered from the side of the scintillator 11 placed at the position represented by the position information P7. Therefore, the position calculator 70 obtains division points J that divide a boundary B1 and a boundary B2 in the depth direction at a ratio of (1:2:0.5), respectively.

Coordinates (X7,Y7) of the upper end and coordinates (X7,Y8) of the lower end of the boundary B1 are defined by the position information P7 and the position information P8. Coordinates (X9,Y9) of the upper end and coordinates (X9,Y10) of the lower end of the boundary B2 are defined by the position information P8 and the position information P9. On each of the boundary B1 and the boundary B2, two division points may exist. However, the position information P110 represent that the gamma ray has entered from the side of the scintillator 11 placed at the position represented by the position information P7. Therefore, on the boundary B1, a division point J (X7,Y11) on the upper end side shall be the division point. On the boundary B2, a division point J (X9,Y12) on the lower end side shall be the division point. Alternatively, either the division point J (X7,Y11) or the division point J (X9,Y12) may be obtained as the division point.

That is to say, the position calculator 70 calculates $Y11 = (2.5{\times}Y7+Y8)/3.5$. Moreover, the position calculator 70 calculates $Y12 = (0.5{\times}Y9+3{\times}Y10)/3.5$.

The position calculator 70 determines either the division point J (X7,Y11) or the division point J (X9,Y12) as the passing position of one gamma ray of paired gamma rays represented by the respective single information of number 07, number 08, and number 09.

Alternatively, the position calculator 70 may determine both the division point J (X7,Y11) and the division point J (X9,Y12) as the passing points of the one gamma ray.

As described above, based on the result that the gamma ray has entered into the plurality of adjacent scintillators 11, the passing position of the gamma ray is calculated. Consequently, it is possible to make the spatial resolution of an image higher than in a conventional PET apparatus while reducing the cost as compared with a DOI detector.

Further, the depthwise thickness of the scintillator 11 is 20 mm or less, preferably 10 mm or less. Consequently, it is easy to let backscatter of Compton scatter occurring within the scintillators 11 outside the scintillators 11. As a result, a fiction that a gamma ray is detected at the same probability at each point on a trajectory passing the position of a radioactive isotope and a calculated passing position, and the result of the detection agree more. Accordingly, the accuracy in calculation of the passing point of the gamma ray increases.

The scintillators 11 convert gamma rays into lights of light amounts corresponding to energies. The detectors 10 are each provided with the position sensitive photomultiplier tubes 14. The position sensitive photomultiplier tubes 14 are optically connected in correspondence with the scintillators 11. The position sensitive photomultiplier tubes 14 convert lights outputted by the respective scintillators 11 into electric signals in accordance with the light amounts. Consequently, it is possible to acquire an output of each of the scintillators 11, and the accuracy in calculation of the passing position of a gamma ray increases.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

**Claims**

1. A positron emission tomography apparatus (1) that generates an image based on a result of detection of a gamma ray radiated from each of radioactive isotopes inside a subject (P), the positron emission tomography apparatus (1) comprising:

   detectors (10) each having a plurality of scintillators (11) each converting the entering gamma ray into a light of a light amount corresponding to an energy of the gamma ray, the detectors (10) being arranged in a ring shape so as to surround the subject (P);
   a position calculator (70) configured to, when the gamma ray has entered into adjacent scintillators of the plurality of scintillators (11) simultaneously in one detector of the detectors (10) and the detector performs detection of the gamma ray having entered simultaneously, calculate a passing position of the gamma ray based on a result of the detection; and
   a reconstructing part configured to execute a back projection process with a trajectory passing through the calculated passing position as a projection direction to reconstruct the image of concentration distribution of the radioactive isotopes inside the subject.

2. The positron emission tomography apparatus (1) according to claim 1, wherein the position calculator (70) is configured to, in a case that the gamma ray has entered into the two adjacent scintillators simultaneously:

   calculate a ratio of wave heights representing energies of the gamma ray detected by the detector;
   calculate such a trajectory of the gamma ray that a ratio of distances passed by the gamma ray within the two scintillators, respectively, coincide with the ratio of the wave heights;
   calculate an intersection between a boundary of the two adjacent scintillators and the trajectory; and
   define the intersection as the passing position.

3. The positron emission tomography apparatus (1) according to claim 1 or 2, wherein the position calculator (70) is configured to, in a case that the gamma ray has entered into the three adjacent scintillators simultaneously:

   calculate a ratio of wave heights representing energies of the gamma ray detected by the detector;
   calculate such a trajectory of the gamma ray that a ratio of distances passed by the gamma ray within the three scintillators, respectively, coincide with the ratio of the wave heights;
calculate an intersection between each of boundaries of the three adjacent scintillators and the trajectory; and
define the intersection on each of the boundaries as the passing position.

4. The positron emission tomography apparatus (1) according to any one of claims 1 to 3, wherein the position calculator (70) is configured to, in a case that the gamma ray has entered into two or three adjacent scintillators simultaneously, calculate the passing position of the gamma ray on a boundary of the two adjacent scintillators or on each of boundaries of the three adjacent scintillators, respectively, based on wave heights representing energies of the gamma ray detected by the detector and stopping power of the two scintillators.

5. The positron emission tomography apparatus (1) according to any one of claims 1 to 4, wherein the position calculator (70) is configured to:

discriminate an entering direction of the gamma ray having entered into the two adjacent scintillators, based on a position of another scintillator into which the gamma ray has entered simultaneously with entrance into the two adjacent scintillators; and
calculate the passing position based on the trajectory of the gamma ray whose direction coincides with the entering direction.

6. The positron emission tomography apparatus (1) according to any one of claims 1 to 5, wherein the position calculator (70) is configured to, in a case that the gamma ray has entered into two or three adjacent scintillators simultaneously:

calculate a ratio of wave heights representing energies of the gamma ray detected by the detector; and
calculate, as the passing position, a position dividing the two scintillators (11) in a depth direction at the ratio on a boundary of the two scintillators, or a position being one point on each of boundaries of the three scintillators, the position dividing the scintillators in a depth direction at the ratio, respectively.

7. The positron emission tomography apparatus (1) according to any one of claims 1 to 6, wherein the position calculator (70) is configured to:

discriminate an entering direction of the gamma ray having entered into the two adjacent scintillators, based on a position of another scintillator into which the gamma ray has entered simultaneously with entrance into the two adjacent scintillators;
obtain a ratio of a wave height representing an energy of the gamma ray having entered into one of the scintillators on a front side of the entering direction and a wave height representing an energy of the gamma ray having entered into the other scintillator on a rear side of the entering direction; and
calculate, as the passing position, a position dividing the scintillators in the depth direction at the ratio.

8. The positron emission tomography apparatus (1) according to any one of claims 1 to 7, wherein a depthwise thickness of at least one of the scintillators (11) is 10 mm or less.

9. The positron emission tomography apparatus (1) according to any one of claims 1 to 8, wherein:

at least one of the detectors (10) is further provided with position sensitive photomultiplier tubes (14);
the position sensitive photomultiplier tubes (14) are optically connected so as to correspond to the individual scintillators (11), and configured to convert a light outputted by each of the scintillators (11) into an electric signal in accordance with an amount of the light; and
at least one of the detectors (10) is configured to output the electric signal as the result of the detection.

10. The positron emission tomography apparatus (1) according to any one of claims 1 to 9, wherein the position calculator (70) is configured to, in a case that a sum of energies of the gamma ray having entered into the adjacent scintillators simultaneously is equivalent to an energy of a Compton edge or more, calculate the passing position based on the result of the detection.

11. A nuclear medical image generating method of performing detection of a gamma ray radiated from each of radioactive isotopes inside a subject (P) with detectors (10) arranged in a ring shape surrounding the subject (P) and each detector having a plurality of scintillators (11), each scintillator converting the entering gamma ray into a light of a light amount corresponding to an energy of the gamma ray, and generating an image based on a result of the detection with the detectors (10), comprising,
when the gamma ray has entered into adjacent scintillators of the plurality of scintillators (11) simultaneously in one detector of the detectors (10) and the detector has performed detection of the gamma ray having entered simultaneously, calculating a passing position of the gamma ray based on a result of the detection; and
reconstructing the image of concentration distribution of the radioactive isotopes inside the subject by execution of a back projection process with a trajectory passing through the calculated passing position as a projection direction.

12. The nuclear medical image generating method according to claim 11, wherein in a case that the gamma ray has entered into two or three adjacent scintillators simultaneously:

a ratio of wave heights representing energies of the gamma ray detected by the detector is calculated;
a trajectory of the gamma ray is calculated such that a ratio of distances passed by the gamma ray within the two or three scintillators, respectively, coincide with the ratio of the wave heights,
an intersection between each of boundaries of the two or three adjacent scintillators and the trajectory is calculated; and
the intersection on each of the boundaries is defined as the passing position.

13. The nuclear medical image generating method according to claim 11 or 12, wherein in a case that the gamma ray has entered into two or three adjacent scintillators simultaneously, the passing position of the gamma ray on each of boundaries of the two or three adjacent scintillators is calculated based on wave heights representing energies of the gamma ray detected by the detector and stopping power of the two or three scintillators.

14. The nuclear medical image generating method according to any one of claims 11 to 13, wherein an entering direction of the gamma ray having entered into the two adjacent scintillators is discriminated based on a position of another scintillator into which the gamma ray has entered simultaneously with entrance into the two adjacent scintillators; and
the passing position is calculated based on the trajectory of the gamma ray whose direction coincides with the entering direction.

15. The nuclear medical image generating method according to any one of claims 11 to 14, wherein in a case that the gamma ray has entered into the two adjacent scintillators simultaneously:

a ratio of wave heights representing energies of the gamma ray detected by the detector is calculated; and
a position dividing the scintillators in a depth direction at the ratio on a boundary of the two scintillators is calculated as the passing position; or wherein in a case that the gamma ray has entered into the three adjacent scintillators simultaneously:

a ratio of wave heights representing energies of the gamma ray detected by the detector is calculated; and
a position being one point on each of boundaries of the three scintillators, the position dividing the scintillators in a depth direction at the ratio, is calculated as the passing position.

# FIG. 1

## PRIOR ART

# FIG. 2

# FIG. 3

| TIME STAMP PART | 30 |

| SINGLE INFORMATION COLLECTOR | 40 |

| SIMULTANEOUSNESS DETERMINING PART | 50 |

| ENERGY DISCRIMINATOR | 60 |

| POSITION CALCULATOR | 70 |

| COINCIDENCE INFORMATION COLLECTOR | 80 |

| IMAGE RECONSTRUCTING PART | 90 |

# FIG. 4

# FIG. 5

J(x0, Y0)

11

e

EP 2 246 712 A2

# FIG. 6

# FIG. 7

# FIG. 8

EP 2 246 712 A2

FIG. 9

# FIG. 10

```
                    ┌─────────────┐
                    │    START    │
                    └──────┬──────┘
(S01)                      │
        ┌──────────────────▼──────────────────┐
        │          DETECT GAMMA RAY           │
        └──────────────────┬──────────────────┘
(S02)                      │
        ┌──────────────────▼──────────────────┐
        │      RECORD SINGLE INFORMATION      │
        └──────────────────┬──────────────────┘
(S03)                      │
        ┌──────────────────▼──────────────────┐
        │     END DETECTION OF GAMMA RAY      │
        └──────────────────┬──────────────────┘
(S04)                      │
        ┌──────────────────▼──────────────────┐
        │            SEARCH GROUP             │
        └──────────────────┬──────────────────┘
(S05)                      │
        ┌──────────────────▼──────────────────┐
        │             SUM ENERGY              │
        └──────────────────┬──────────────────┘
(S06)                      │
        ┌──────────────────▼──────────────────┐
        │     DETERMINE WHETHER SUM IS        │
        │  INCLUDED WITHIN A RANGE SHOWN      │
        │   BY ENERGY RANGE INFORMATION       │
        └──────────────────┬──────────────────┘
(S07)                      │
        ┌──────────────────▼──────────────────┐
        │   DIVIDE GROUP INTO SMALL GROUPS    │
        └──────────────────┬──────────────────┘
                           │
                        ┌──▼──┐
                        │  A  │
                        └─────┘
```

# FIG. 11

(A)

(S08) SMALL GROUP CONTAIN PLURAL SINGLE INFORMATION? — NO

↓ YES

(S10) DETERMINE INCIDENT DIRECTION OF GAMMA RAY

(S11) CALCULATE RATIO OF PEAK VALUE

(S12) OBTAIN DIVISION POINT

(S09) DETERMINE COORDINATE INFORMATION CORRESPONDING TO POSITION INFORMATION AS PASSING POSITION

(S13) SMALL GROUP CONTAIN THREE OR MORE SINGLE INFORMATION? — NO

↓ YES

(S14) DETERMINE REPRESENTATIVE DIVISION POINT AS PASSING POSITION

(S15) GENERATE COINCIDENCE INFORMATION

(S16) RECONSTRUCT IMAGE BASED ON COINCIDENCE INFORMATION

END

# FIG. 12

| SINGLE INFORMATION LIST | | | |
|---|---|---|---|
| NUMBER | POSITION INFORMATION | DETECTION TIMING INFORMATION | ENERGY INFORMATION |
| 01 | P1 | $\fallingdotseq$ TI | E |
| 02 | P2 | $\fallingdotseq$ TI | 2E |
| 03 | P100 | $\fallingdotseq$ TI | 3E |
| 04 | P4 | $\fallingdotseq$ T2 | E |
| 05 | P5 | $\fallingdotseq$ T2 | 2E |
| 06 | P30 | $\fallingdotseq$ T2 | 3E |
| 07 | P7 | $\fallingdotseq$ T3 | E |
| 08 | P8 | $\fallingdotseq$ T3 | 2E |
| 09 | P9 | $\fallingdotseq$ T3 | 0.5E |
| 10 | P110 | = T3 | 3E |

**EP 2 246 712 A2**

**REFERENCES CITED IN THE DESCRIPTION**

**Patent documents cited in the description**

- JP 2005090979 A